# EUROPEAN PATENT SPECIFICATION

(11) **EP 4 019 942 B1**
(45) Date of publication and mention of the grant of the patent: **21.06.2023**
(21) Application number: 20856652.1
(22) Date of filing: 14.08.2020
(51) Int. Cl.: G01N 21/3563

(54) **BIOLOGICAL TISSUE IDENTIFICATION METHOD AND BIOLOGICAL TISSUE IDENTIFICATION DEVICE**
VERFAHREN UND VORRICHTUNG ZUR IDENTIFIZIERUNG VON BIOLOGISCHEM GEWEBE
MÉTHODE ETDISPOSITIF D'IDENTIFICATION DE TISSU BIOLOGIQUE

(30) Priority: 23.08.2019 JP 2019153286
(43) Date of publication of application: 29.06.2022
(73) Proprietor: Light Touch Technology Incorporated, Osaka-shi, Osaka, 540-0029 (JP)
(72) Inventor: YAMAKAWA, Koichi, Osaka-shi, Osaka 540-0029 (JP); AOYAMA, Makoto, Osaka-shi, Osaka 540-0029 (JP); OGAWA, Kanade, Osaka-shi, Osaka 540-0029 (JP)
(74) Representative: Hoffmann Eitle
(86) International application number: PCT/JP2020/030887
(87) International publication number: WO 2021/039441

(56) References cited:
- WO-A1-2013/063316
- WO-A1-2013/099141
- WO-A1-2014/196097
- WO-A1-2015/107722
- WO-A1-2016/170656
- JP-A- 2004 135 868
- JP-A- 2007 218 749
- JP-A- 2011 015 163
- JP-A- 2011 239 195
- JP-A- 2014 531 065
- JP-A- 2017 027 138
- US-A1- 2004 181 375
- CASE, JASON R. et al.: "Noninvasive enhanced mid- IR imaging of breast cancer development in vivo", Journal of Biomedical Optics, vol. 20, no. 11, 2015, pages 116003-1-116003-9, XP060071850, DOI: 10.1117/1.JBO.20.11.116003

## Description

### TECHNICAL FIELD

The present invention relates to a method for identifying a biological tissue using mid-infrared light, and a biological tissue identification apparatus. The present invention also relates to a program for causing a biological tissue identification apparatus to execute processing.

### BACKGROUND ART

For identification of the type or state of a biological tissue, for example, identification of the existence or non-existence of cancer or the like, a histopathological method is used in which a tissue taken from a living body (subject) is stained with a dye, and observed with a light microscope to make a diagnosis by a pathologist. This method has the problem that time and effort are required for diagnosis, and a diagnosis result heavily depends on the judgment of a pathologist.

As a method which enables a biological tissue identified in a shorter time as compared to a histopathological method, an imaging technique using mid-infrared light has attracted attention. The vibration frequency of mid-infrared light coincides with the natural vibration frequency of a substance, and the mid-infrared light is characterized by being absorbed by resonance with molecular vibration of the substance, so that it is possible to identify whether the test target is a normal tissue or a tissue including cancer, etc. on the basis of an absorption spectrum in a mid-infrared region. In imaging using mid-infrared light, an absorption spectrum is measured in a plurality of microscopic regions within a photographed region, and a set of spectra (hyperspectra) in each microregion (pixel) is analyzed by a predetermined algorithm to obtain an objective identification result that does not depend on a subjective view of an examiner.

Patent Document 1 discloses a technique which enables a wide area of a biological tissue to be imaged at a time by using a two-dimensional array type sensor camera having sensitivity in a mid-infrared region. In particular, Patent Document 1 discloses an imaging microscope working in the spectral region of 2 µm to 20 µm for analyzing a tissue sample using both reflected and transmitted infrared radiation.

In Non-Patent Document 1, a quantum cascade laser (QCL) with a high light intensity is used as an irradiation light source of middle infrared light to perform measurement in a short time.

### PRIORART DOCUMENT

### PATENT DOCUMENT

Patent Document 1: WO 2013/063316

### NON-PATENT DOCUMENT

Non-Patent Document 1: Kroger et al., Journal of Biomedical Optics. 2014, 19 (11), 111607.

### SUMMARY OF THE INVENTION

### PROBLEMS TO BE SOLVED BY THE INVENTION

In a conventional imaging method using mid-infrared light, multivariate analysis such as cluster analysis is performed on a hyperspectral image of a biological tissue to identify the biological tissue from a tissue image displayed as a color map. This method enables identification in a short time as compared to histopathological diagnosis, but is difficult to apply to identification of a biological tissue in real time because the amount of data processed is large, so that much time is required for analysis.

In view of such circumstances, an object of an aspect of the present invention is to provide a biological imaging method which enables a biological tissue to be identified in a shorter time.

### MEANS FOR SOLVING THE PROBLEMS

The present invention is defined in independent claims 1 and 8.

One embodiment of the present invention relates to a method including irradiating a predetermined region of a biological tissue with measurement light, acquiring a measurement image by imaging measurement light transmitted through the biological tissue or measurement light reflected at the biological tissue, and identifying the biological tissue based on a result of analysis of the measurement image. The measurement light is light including mid-infrared light, and may be coherent light or incoherent light.

A measurement image is acquired by imaging a part of measurement light transmitted through the biological tissue or measurement light reflected at the biological tissue, which has a specific wavelength λ₁ being a wavelength in the range of 2 µm to 20 µm. When the measurement light is coherent light, the biological tissue may be irradiated with light having a wavelength λ₁ as measurement light. When the measurement light is incoherent light, a part of measurement light, which has a wavelength λ₁, may be imaged by using an appropriate spectroscopic unit.

By analyzing the measurement image, a focusing measure is calculated, and a biological tissue is identified on the basis of the focusing measure. The focusing measure is calculated by edge analysis of a measurement image. For example, edge analysis is performed by differentiation filtering of a measurement image.

The identification of the living tissue is, for example, identification of whether or not cancer or the like is contained in a biological tissue. For example, whether or not the biological tissue is a normal tissue (whether or not a tumor is present) is identified on the basis of a magnitude relationship between a focusing measure and a predetermined threshold.

One aspect of the present invention is a biological tissue identification apparatus for carrying out the method for identifying a biological tissue. The biological tissue identification apparatus includes a light source capable of applying measurement light, and an imaging element for acquiring a measurement image by imaging measurement light transmitted through a biological tissue or measurement light reflected at the biological tissue, in a predetermined region.

The biological tissue identification apparatus further includes a computer including a calculation analysis unit for analyzing a measurement image to calculate a focusing measure. A storage unit of a computer may store a program for causing a calculation analysis unit to execute processing in which the measurement image acquired by the imaging element is subjected to edge analysis to calculate focusing measures in a plurality of regions of the measurement image. The computer may include an image forming unit for mapping the focus measures to form an analysis image.

The light (measurement light) applied from the light source to the biological tissue is preferably coherent light. The light source for irradiating the biological tissue with coherent light as measurement light is, for example, a laser light source capable of scanning wavelengths, and a quantum cascade laser or a laser including an optical parametric oscillator is suitable.

In an aspect of the present invention, focusing adjustment is performed before acquirement of the measurement image. A focusing function which is an index of a focusing degree is calculated for light having a wavelength λ₀ different from the wavelength λ₁ while the positional relationship between the optical elements is changed, and the positional relationship between the optical elements is adjusted so that the focusing function becomes maximum or minimum (the focusing degree becomes maximum). In one embodiment, the position of a sample stage with a sample fixed thereon is changed, and the measurement and the calculation of the focusing function are performed at the position of each sample stage.

One aspect of the present invention is a biological tissue identification program. A processing procedure for causing the computer to execute each processing described above. The biological tissue identification program may be provided as a readable storage medium which can be read by a computer.

### EFFECTS OF THE INVENTION

In the method of the present invention, a biological tissue is irradiated with mid-infrared measurement light, and a measurement image obtained by imaging light having a specific wavelength is subjected to edge analysis. On the basis of the resulting focusing measure, a biological tissue can be identified for existence or non-existence of cancer. This method enables data processing to be performed in a short time, and therefore can be expected to be applied to real-time analysis.

### BRIEF DESCRIPTION OF THE DRAWINGS

FIG. 1 is a block diagram of a biological tissue identification apparatus according to an embodiment.
FIG. 2 is a flow chart showing an example of a flow of processing in a biological tissue identification apparatus.
FIG. 3 is a pathological image obtained by staining a lung-derived sample containing a normal tissue and a cancer tissue, and imaging the sample with an optical microscope.
FIG. 4 is an analysis image obtained by mid-infrared imaging of a lung-derived sample (unstained) containing a normal tissue and a cancer tissue.
FIG. 5 is a pathological image obtained by staining a sample containing a normal tissue and a bone sarcoma, and imaging the sample with an optical microscope.
FIG. 6 is an analysis image obtained by mid-infrared imaging of a sample (unstained) containing a normal tissue and a bone sarcoma.
FIG. 7 is a pathological image obtained by staining a liver-derived sample containing a normal tissue and a cancer tissue, and imaging the sample with an optical microscope.
FIG. 8 is an analysis image obtained by mid-infrared imaging of a liver-derived sample (unstained) containing a normal tissue and a cancer tissue.

### MODE FOR CARRYING OUT THE INVENTION

Hereinafter, an embodiment of the present invention will be described with reference to the drawings. The present invention is not limited to the following specific embodiment.

### [Configuration of biological tissue identification apparatus]

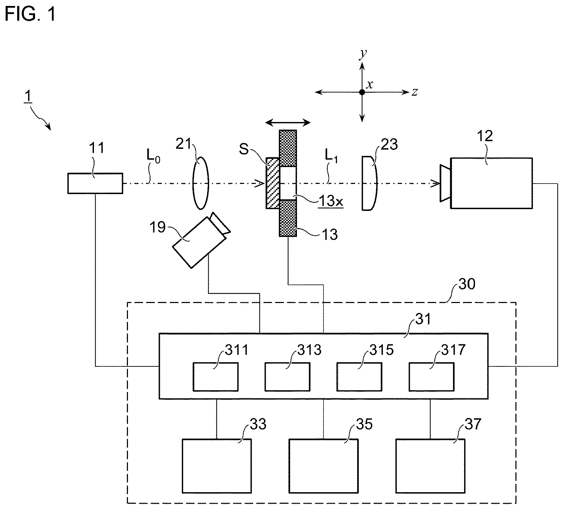

FIG. 1 is a block diagram of a biological tissue identification apparatus according to an embodiment. In a biological tissue identification apparatus 1, a sample S fixed on a sample stage 13 is irradiated with measurement light (mid-infrared light) L₀ from a light source 11, and measurement light L₁ transmitted through the sample S is imaged by an imaging element 12.

The light source 11, the sample stage 13, and the imaging element 12 are connected to a computer 30. The computer 30 includes a control unit 31 which controls adjustment of the power of light source 11, adjustment of the position of the sample stage 13, acquisition and analysis of information from the imaging element 12, and the like. A measurement image obtained by the imaging element 12 is analyzed by the computer 30 to identify the biological tissue contained in the sample S.

The computer 30 includes a display unit 33, a storage unit 35 and an input unit 37 in addition to the control unit 31. The display unit 33 is a display which displays various kinds of information and measurement results. The storage unit 35 includes a hard disk or a semiconductor memory which stores various kinds of data. The input unit 37 includes a touch panel, a keyboard, a mouse and the like for receiving user's operations.

In the apparatus 1 shown in FIG. 1, the sample stage 13 is disposed between the light source 11 and the imaging element 12 for imaging the measurement light L₁ transmitted through the sample S by the imaging element 12. When measurement light reflected at the sample is imaged, the imaging element may be disposed on the light source side of the sample stage.

The measurement light L₀ applied from the light source 11 to the sample S is light including mid-infrared light (wavelength: 2 µm to 20 µm). The measurement light L₀ may be coherent light or incoherent light. When the measurement light is coherent light, the measurement light L₀ is light having a specific wavelength in the mid-infrared region. When the measurement light is incoherent light, the measurement light L₀ may include light having a wavelength which is not in the mid-infrared region.

The measurement light L₀ is preferably coherent light because the light intensity is high at a specific wavelength, so that the measurement time can be shortened. The light source 11 which irradiates the sample with coherent light as measurement light is, for example, a laser light source which emits mid-infrared light, and a laser light source capable of scanning wavelengths in the mid-infrared region is preferable. From the viewpoint of improving measurement accuracy and shortening the measurement time, it is preferable to use a quantum cascade laser (QCL) which emits a high-luminance mid-infrared laser beam or a light source including an optical parametric oscillator (OPO). Details of the OPO are described in, for example, Japanese Patent Application Laid-open No. 2010 -281891.

As an excitation light source of the OPO, a laser capable of emitting excitation light which is pulsed and has a wavelength shorter than that of mid-infrared light is used, and a Q-switched Nd: YAG laser (emission wavelength: 1.064 µm) or a Q-switched Yb: YAG laser (emission wavelength: 1.030 µm) is particularly preferably used. Such an excitation light source allows switching operations to be automatically performed using an oversaturated absorber. Thus, by using a Q-switched Nd: YAG laser or a Q-switched Yb: YAG laser as an excitation light source, the excitation light source and the configuration for control thereof can be simplified and downsized. In the Q-switch oscillation, excitation light can be emitted with a pulse width of about 8 ns and repetition of 10 Hz or more.

In the OPO, a nonlinear optical crystal is installed between semi-transparent mirrors on the incident side and on the reflection side. As the nonlinear optical crystal, for example, AgGaS₂ is used. Examples of other nonlinear optical crystals include GaSe, ZnGeP₂, CdSiP₂, LiInS₂, LiGaSe₂, LiInSe₂ and LiGaTe₂. The laser light emitted from the OPO 12 has a repetition frequency and a pulse width (e.g. about 8 ns) which correspond to the excitation light, and because of the short pulse width, the intensity of the peak power is as high as 10 W to 1 kW.

The light source 11 may be configured such that the amount of light and the emission wavelength can be adjusted on the basis of a signal from a light adjustment unit 311 of the control unit 31. For example, by adjusting the power of the laser, the amount of light with which the sample S is irradiated can be adjusted. The amount of light may be adjusted by adjustment of a pulse width and a beam size of a laser, use of an optical filter, adjustment of a shutter speed, adjustment of an integrated time and a gain of an imaging element, and the like.

An optical element 21 such as a lens may be disposed between the light source 11 and the sample stage 13 for adjusting the size (light diameter) of irradiation light. The optical element 21 may increase or decrease the diameter of light applied to the sample S. The optical element 21 may be configured to be movable on the basis of a signal from a position adjustment unit 313 of the control unit 31. By moving the optical element 21 in parallel with the traveling direction of laser light (z direction), adjustment of the light diameter of light applied to the sample S and adjustment of a focus (focusing adjustment) can be performed.

The sample stage 13 is capable of fixing the sample S. For example, the sample S is placed on the sample stage 13 to fix the sample S. The sample S may be fixed to the sample stage 13 by using a clip or the like. The biological tissue contained in the sample S may be a tissue taken from a human or a tissue taken from a non-human animal such as a mouse or a rat.

The sample stage 13 can transmit the measurement light L₁ transmitted through the sample S. For example, in the sample stage 13, a portion on which the sample S is placed may be formed of a material transparent to mid-infrared light. As shown in FIG. 1, the sample stage 13 may be provided with an opening 13x for transmitting the measurement light L₁.

The sample stage 13 may be configured to be movable in parallel with the traveling direction of the measurement light (middle infrared laser light) L₀ and L₁ (z direction in FIG. 1). By moving the sample stage 13 along the traveling direction of measurement light, the distance between the light source 11 and the sample S (and the distance between the optical element 21 and the sample S) and the distance between the sample S and the imaging element 12 (and the distance between the sample S and the optical element 23) can be changed to perform focusing adjustment. The movement (adjustment of the position) of the sample stage may be performed manually or on the basis of a signal from the position adjustment unit 313 of the control unit 31.

The sample stage 13 may be configured to be movable two-dimensionally in directions (x direction and y direction) orthogonal to the traveling direction of measurement light. When the sample stage is configured to be movable in the xy directions, the position at which the measurement light L₀ is applied in the sample S can be adjusted.

The measurement light L₁ transmitted through the sample S is detected by the imaging element 12. The imaging element 12 has a plurality of pixels arranged two-dimensionally (in the x direction and the y direction), and detects the intensity of the measurement light L₁ incident to each pixel. Data of the light intensity incident to each pixel is sent to the computer 30, and a measurement image is formed in an image forming unit 317 of the control unit 31. The measurement image is formed on the basis of the luminance (integrated amount) of the measurement light L₁ incident to each pixel of the imaging element 12. That is, the measurement image is obtained by mapping the intensity of transmitted light incident to each pixel.

A calculation analysis unit 315 of the control unit 31 analyzes the measurement image. The image forming unit 317 forms an analysis image on the basis of the result of analysis. The formed analysis image is displayed on the display unit 33.

An imaging optical element 23 for adjusting the light diameter of the measurement light L₁ reaching the imaging element 12 may be disposed between the sample stage 13 and the imaging element 12. In general, the light receiving area of the imaging element 12 is larger than the area of a region in which the sample S is irradiated with the measurement light L₀. When the light diameter of the measurement light L₁ by the imaging optical element 23 and the measurement light is made to enter the imaging element 12, so that a wider region of the imaging element is used, the resolution of the image can be enhanced.

The biological tissue identification apparatus 1 may include a second imaging element 19 for directly imaging the sample S in addition to the imaging element 12 for detecting the measurement light L₁ transmitted through the sample S (or measurement light reflected by the sample S). The second imaging element 19 is disposed on the light source 11 side when viewed from the sample S, and is capable of imaging a region including a portion irradiated with the measurement light L₀ in the sample S. An appropriate optical element (not shown) may be disposed between the sample S and the second imaging element 19. An image of the sample S imaged by the second imaging element 19 may be displayed on the display unit 33. The user may adjust the position at which the measurement light L₀ is applied in the sample S while observing the image on the display unit. Since the measurement light in the mid-infrared region cannot be observed by the imaging element 19 for visible light, the sample S may be irradiated with visible light from a light source (not shown) in adjustment of the position at which the light is applied.

### [Measurement and imaging of biological tissue]

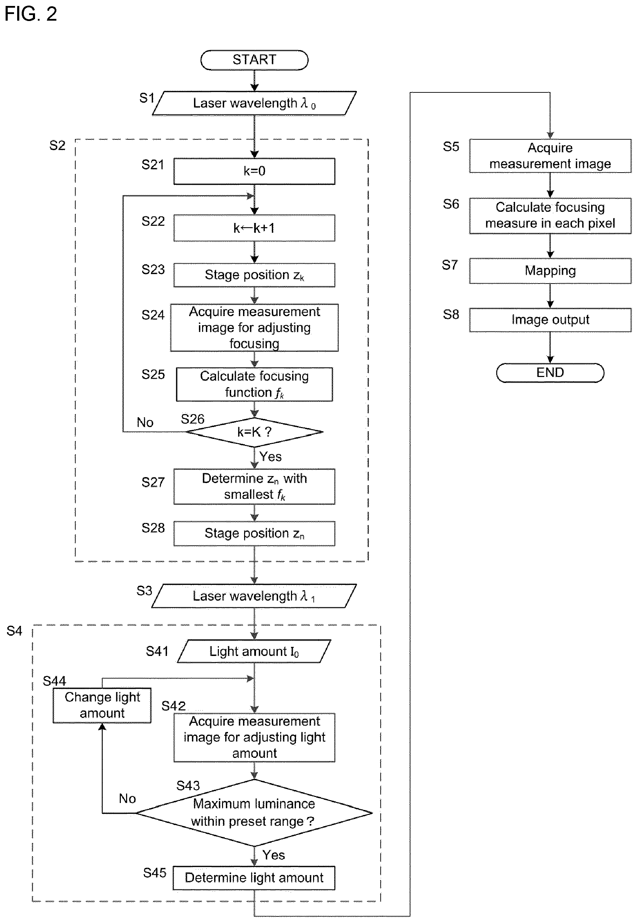

FIG. 2 is a flow chart showing an example of processing by the biological tissue identification apparatus 1. First, the wavelength of laser light applied from the light source 11 to the sample S is set to a focusing adjusting wavelength λ₀ (S1), and focusing adjustment is performed with mid-infrared light having a wavelength λ₀ (S2). After the focusing adjustment, the wavelength of the laser light is changed to a measuring wavelength λ₁ (S3), and the amount of light is adjusted with the mid-infrared light having a wavelength λ₁ as necessary (S4). Thereafter, the sample S is irradiated with the mid-infrared light having a wavelength λ₁, and a measurement image is acquired by the imaging element 12 (S5). The obtained measurement image is analyzed (S6), the results of the analysis are mapped to form an image (analysis image) (S7), and the analysis image is displayed on the display unit 33 (S7). On the basis of the obtained analysis image, the existence or non-existence of a diseased tissue and a location thereof are identified.

Each processing described above is controlled by a control block (light adjustment unit 311, position adjustment unit 313, calculation analysis unit 315 and image forming unit 317) of the control unit 31. A biological tissue identification program which is software for executing the above-mentioned processing may be stored in the storage unit 35 of the computer 30. The control unit 31 includes one or more processors, and the processor reads the biological tissue identification program from the storage unit 35, and each control block operates each element of the biological tissue identification apparatus to execute processing.

The biological tissue identification program may be provided together with the biological identification apparatus in a state of being stored in the storage unit 35 of the computer 30. The biological tissue identification program may be provided in a state of being stored in a readable storage medium which can be read by a computer, or the biological tissue identification program may be provided from an external server or the like through a wired or wireless network. A program stored in a readable storage medium, an external server or the like may be stored in the storage unit 35 of the computer 30 and used.

### < Example 1: Measurement of lung sample >

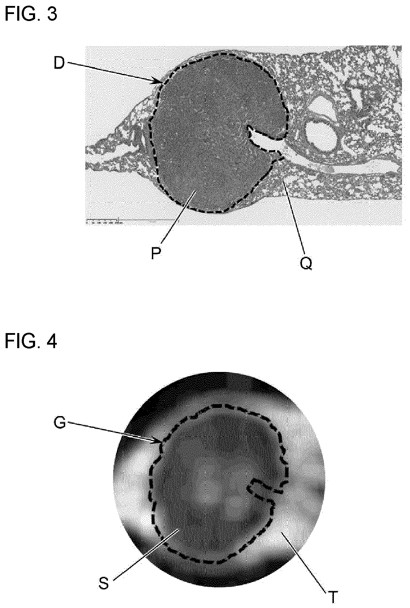

FIG. 3 is a pathological image obtained by staining a lung sample containing a normal tissue and a cancer tissue, and imaging the sample with an optical microscope. A region P surrounded by a broken line D in the drawing is a region including a cancer tissue, and another region Q is a normal tissue. FIG. 4 shows an analysis image obtained by providing a lung sample (unstained) containing a normal tissue and a cancer tissue as a sample, irradiating the sample with laser light having a wavelength of 9.6 µm, analyzing a target region from the obtained measurement image, and mapping focusing measures of pixels in 20 gradations. The focusing measure was calculated by edge analysis using a Sobel filter which is a type of differentiation filter.

The position of a broken line G in FIG. 4 corresponds to the position of the broken line D in FIG. 3, a region S surrounded by the broken line G corresponds to the region P including cancer, and an outside region T corresponds to a region Q of the normal tissue. Since in FIG. 4, the region S has a small focusing measure and the region T has a large focusing measure, it can be seen that the magnitude of the focusing measure corresponds to the existence or non-existence of cancer.

Hereinafter, with reference to the flowchart of FIG. 2, a flow of processing of an example is described in which a lung sample containing a normal tissue and a cancer tissue was provided as a sample, the image of FIG. 4 was acquired by a biological tissue identification apparatus 1, and a biological tissue was identified.

In this example, a sample S placed on a sample stage 13 was irradiated with a mid-infrared laser light having a pulse width of 8 ns and a measuring wavelength λ₁ of 9.6 µm from a light source 11 obtained by combining a Q-switched Nd: YAG laser (emission wavelength: 1.064 µm) with an optical parametric oscillator using AgGaS₂ as a nonlinear optical crystal, and light transmitted through the sample was photographed by an imaging element 12. As the imaging element 12, a two-dimensional bolometer array sensor (14 bits) having 480 × 640 pixels was used. Lenses were disposed as optical elements 21 and 23 between the light source 11 and the sample stage 13 and between the sample stage 13 and the imaging element 12, respectively.

### (Selection of focusing adjusting wavelength)

First, the wavelength of mid-infrared light applied from the light source 11 to the sample S is set to a focusing adjusting wavelength λ₀ (S1). The wavelength of laser light emitted by the light source 11 can be changed on the basis of a signal from a light adjustment unit 311. In this embodiment, the focusing adjusting wavelength λ₀ was set to 11 µm, but may be another wavelength, and a wavelength at which a difference in light absorption between a normal tissue and a diseased tissue (e.g. cancer) is small may be selected.

Depending on the type or state of the biological tissue (organ), there is a range of wavelengths at which the absorbance hardly changes. For example, a wavelength (isosbestic point) at which the absorbance does not change even when the state of the tissue changes is present at or near a peak of an absorption spectrum. Even when a normal tissue and a diseased tissue are mixed, a wavelength at or near the isosbestic point is suitable as a focusing adjusting wavelength because a difference in light absorption between a normal tissue and a diseased tissue is small. The focusing adjusting wavelength may be a wavelength corresponding to a valley of the absorption spectrum or a wavelength corresponding to a flat portion of the absorption spectrum.

### (Focusing adjustment)

A region to be diagnosed may be selected from a formed image (a region where the sample is irradiated with mid-infrared light), followed by performing the focusing adjustment and the analysis of a measurement image on the selected region. In this example, the circular region shown in FIG. 4 was defined as the target region.

First, the position of the sample stage 13 in a z direction is adjusted to an initial position z₁ (S21 to 23), the sample S is irradiated with mid-infrared laser light having a wavelength λ₀ (11 µm in this example), and an image of light transmitted through the sample is formed by the imaging element 12 to acquire a focusing adjusting image A₁ (S24). The measurement image is formed on the integrated amount of light incident to each pixel of the imaging element 12. The focusing adjusting image A₁ is analyzed to calculate a focusing function f₁ as an index of focusing (S25).

Thereafter, the sample stage 13 is moved in the z direction to be adjusted to a position z₂ (S26, S22 and S23), the focusing adjusting image A₂ is acquired (S24), and the focusing function f₂ is calculated (S25). While the distance between the lens 23 and the sample S is changed by changing the position of the sample stage, a focusing adjusting image Aₖ is acquired at each of the positions zₖ (k is an integer of 1 to K) of the sample stage, and a focusing function fₖ, which is an evaluation value representing the degree of focusing, is calculated. A position zₖ at which the focusing function fₖ is smallest (or largest) is defined as a focusing position zₙ (S27), and the stage is moved to the focusing position zₙ (S28).

In this example, focus adjustment was performed while the sample stage 13 was moved, but the lens 23 may be moved in the z direction instead of moving the sample stage. Both the lens 23 and the sample stage 13 may be moved in the z direction, or the light source 11, the lens 23 and the imaging element 12 may be moved in the z direction.

In this example, edge analysis is performed with a Sobel filter, which is a type of differentiation filter, applied to the focusing adjustment image Aₖ, a differential value of each pixel in the target region is defined as a focus measure, and a difference between the minimum and the maximum of the focus measure in the target region is defined as a focusing function fₖ.

In the Sobel method, differential values between the light intensity (luminance) of one center pixel and the light intensities of eight pixels adjacent to the center pixel are calculated, and differential values with respect to the eight pixels are leveled to obtain a differential value for the center pixel. In the leveling, differential values with respect to four pixels located above and below the center pixel and on the right and left of the center pixel are weighted. By weighting and leveling differential values for the upper, lower, left and right pixels, the accuracy of edge detection is improved. The differential value is calculated for all the pixels in the target region, and a difference between the maximum and the minimum of the differential value in the target region is defined as the focusing function fₖ. It can be determined that the focusing was optimized at the position zₖ where the focusing function fₖ was the smallest.

The differentiation filter used for edge detection is not limited to a Sobel filter, and various primary differentiation filters such as a Prewitt filter and a Roberts filter may be used, or secondary differentiation filters may be used. Edge detection may be performed with a differentiation filter combined with other processing (e.g. noise reduction processing by smoothing).

In the edge detection, a focusing function may be calculated by image analysis using a frequency filter for Fourier transformation, wavelet transform or the like instead of a differentiation method. It is also possible to calculate a focusing measure on the basis of the dispersion of the luminance, and as an example, and as an example, the measurement image is divided into a plurality of regions, and the dispersion of the luminance in each region is used as a focusing measure. Since the dispersion of the luminance is large in a focusing image and the dispersion of the luminance is small in a non-focusing image, it can be determined that a region in which the dispersion is larger is more intensively focused. For focusing adjustment, various focusing adjustment methods used in imaging apparatuses for wavelengths other than mid-infrared wavelengths (e.g. visible light) can also be used.

### (Selection of measuring wavelength)

After focusing adjustment, the wavelength of mid-infrared light applied from the light source 11 to the sample S is changed to a focusing adjusting wavelength λ₁ (S3). In this example, the measuring wavelength λ₁ was set to 9.6 µm, but may be another wavelength. As described above, a wavelength at which a difference in light absorption (absorbance) between a normal tissue and a diseased tissue is large is present in the mid-infrared region. Such a wavelength at which the difference in absorbance may be selected as the measurement wavelength λ₁ according to the type of tissue.

As the wavelength λ₀ used for focus adjustment and the wavelength λ₁ used for measurement, optimum values corresponding to the type of tissue (organ) to be identified are present. The values of the wavelengths λ₀ and λ₁ may be input from the input unit 37 by the user, or selected from candidates displayed on the display unit. For various organs, an appropriate focusing wavelength λ₀ and measurement wavelength λ₁ may be stored in the storage unit 35, followed by reading wavelengths λ₀ and λ₁ correspond to a tissue to be identified, which are input or selected by the user, from the storage unit. Instead of storing optimum wavelengths in the storage unit in advance, the wavelengths λ₀ and λ₁ may be received from an externally accumulated database through a wired or wireless network. The information of wavelengths λ₀ and λ₁ received through the network may be stored in the storage unit 35 on the temporary basis, and the wavelengths stored in the storage unit may be read according to the type of organ during focusing and measurement.

### (Adjustment of amount of light)

Adjustment of the amount of light may be performed (S4) after the wavelength of light emitted from the light source 11 is changed to the measuring wavelength λ₁ and before the measurement is performed. The amount of light can be changed on the basis of a signal from a light adjustment unit 311. When the amount of light is optimized and the dynamic range of the imaging element is used over a range as wide as possible, the contrast of an image of light transmitted through the sample S is enhanced, so that the accuracy of analysis is improved, and accordingly, tissue identification accuracy can be improved.

In the adjustment of the amount of light, first, the amount of light is set to the initial amount of light I₀ (S41). The sample S is irradiated with the measurement light L₀ having a wavelength λ₁, the imaging element 12 receives the measurement light L₁ transmitted through the sample S, and a measurement image B₀ for adjustment of the amount of light is acquired (S42).

Whether or not the maximum luminance (number of counts) in the measurement image B₀ is within a preset range is determined (S43). It is preferable that the maximum luminance is set within a range of highest possible luminances as long as the detector of the imaging element 12 is not saturated, and the maximum luminance may be set within a range of, for example, about 55 to 95% of the maximum number of counts the imaging element.

When the maximum of the luminance of the measurement image for adjustment of the amount of light is outside the set range, the amount of light is changed (S44), the image for adjustment of the amount of light is acquired with the changed amount of light (S42), this process is repeated until the maximum of the luminance falls within the set range, and the amount of light for measurement is determined (S45). In this example, the number of pulses of the laser was adjusted so that the maximum luminance of the measurement image was about 10000 counts with respect to a dynamic range of 14 bits (maximum 16383 counts).

After the maximum of the luminance falls within the set range, the amount of light for bringing the maximum of the luminance close to the optimum value within the set range may be calculated, and defined as the amount of light for measurement. Instead of repeatedly changing the amount of light and acquiring the measurement image for adjustment of the amount of light, the optimum amount of light for measurement may be calculated on the basis of the ratio of the set value to the maximum of the luminance in the measurement image B₀ for adjustment of the amount of light which is measured with the initial amount of light I₀.

### (Imaging)

Focusing adjustment is performed at the wavelength λ₀ (S1, S2), the wavelength is changed to λ₁ (S3), the amount light is adjusted (S4) as necessary, and main measurement is then performed to acquire a measurement image C (S5). In the main measurement, the sample S is irradiated with the measurement light L₀, and the measurement light L₁ having a wavelength λ₁ transmitted through the sample S is imaged by the imaging element 12.

### (Analysis)

The measurement image C acquired in the main measurement is analyzed, and the focusing measure in each pixel of the target region is calculated (S6). In this example, similarly to the focusing adjustment, edge analysis of a measurement image was performed by applying a Sobel filter, and a differential value of each pixel was defined as a focusing measure.

In this example, edge analysis was performed by a Sobel method, and the result showed that the minimum and the maximum of the focusing measure (differential value) in the target region were 130 and 187, respectively. The smaller the differential value, the lower the degree of focusing. The larger the differential value, the higher the degree of focusing.

For the edge analysis, a method may be adopted which ensures that a focus measure as an index of the degree of focus can be calculated for each pixel, and the edge analysis is not limited to the Sobel method. For example, various primary differentiation filters such as a Prewitt filter and a Roberts filter, or secondary differentiation filters may be used. Instead of the differentiation method, a frequency filter such as a Fourier transform or a wavelet transform may be applied to calculate the focusing measure of each pixel. When the resolution of the measurement image is sufficiently large, edge analysis may be performed with the resolution reduced by leveling a plurality of pixels into one region.

### (Mapping and output of result)

The image forming unit 317 maps and images the focusing measure of each pixel obtained by the edge analysis (S7). In this example, an image was formed by dividing a range from the minimum 130 to the maximum 187 of the focusing measure into 20 gradations (see FIG. 4). An image formed by the image forming unit 317 is displayed on the display unit 33 (S8).

In this example, the position of the middle gradation (tenth gradation: 47 to 52%) between the minimum (first gradation) and the maximum (twentieth gradation) of the focusing measure was confirmed to coincide with the boundary between the normal tissue and the cancer tissue in the pathological image of FIG. 3 in the analysis image of FIG. 4. In FIG. 4, the boundary between the normal tissue and the cancer tissue is marked with a broken line on the basis of a correspondence relationship with the pathological image of FIG. 3. The focusing measure of the portion marked with a broken line ranged from 157 to 166, and these values all fell within the range of the tenth gradation in the analysis image of 20 gradations.

The region S surrounded by a broken line G in FIG. 4 is a region having ninth and lower gradations (focusing measure: 156 or less) and a focusing measure smaller than a threshold value and including cancer. The region T outside the broken line is a normal tissue region having eleventh and higher gradations (focusing measure: 167 or more) and a focusing measure larger than a threshold. That is, a region having higher gradations (eleventh to twentieth gradations) over the boundary line is determined as a normal tissue, and a region having lower gradations (first to ninth gradations) is determined as cancer. In this example, a low-gradation region having a small focusing measure is a region including cancer, and a high-gradation region having a large focusing measure is a normal tissue.

In this example, for showing the correspondence relationship between the pathological image (FIG. 3) and the map of the focusing measure (FIG. 4), a sample in which a cancer tissue and a normal tissue are known on the basis of the pathological image is used, and a position corresponding to the boundary line D in the pathological image of FIG. 3 is marked with a broken line G. In measurement of a sample in which a pathologic region is unknown, a boundary of a gradation as a threshold may be marked with a boundary line during outputting of an image. By marking a gradationally divided image with a boundary line, a tissue can be more simply and easily identified without depending on a subjective view of an examiner.

In this example, an image was formed by dividing a range from the minimum to the maximum of the focusing measure into 20 gradations, but the number of gradations in the analysis image may be 20 or more, or may be 2 or more and 19 or less. For example, an image may be displayed in two gradations where the focusing measure is equal to or greater than (or greater than) the threshold and the focus measure is less than (or equal to or less than) the threshold. In this example, the analysis image was displayed in 20 gradations in monochrome, but the image may be colored.

An image of the sample S imaged by the second imaging element 19 may be displayed together with the analysis image on the display unit 33. By contrasting the image of the sample S with the analysis image, the measurement point (diagnosis target portion) in the sample S can be visually recognized. The analysis image may be superimposed on the image of the sample S formed by the second imaging element 19.

### <Principle and advantage of identification of tissue>

As described above, in this example, the sample S was irradiated with measurement light, a transmitted light image having a specific wavelength λ₁ was formed, and a normal tissue and a cancer tissue were identified on the basis of the magnitude of the focusing measure (differential value) determined by edge analysis of the obtained measurement image. In the lung sample, it can be determined that the tissue is a normal tissue when the differential value is large (the degree of focusing is high), and it can be determined that the tissue is a cancer tissue when the differential value is small (the degree of focusing is low). This is based on the finding that when a biological tissue is irradiated with mid-infrared light having a predetermined wavelength, the contrast of the image of a normal tissue differs from that of a diseased tissue such as cancer, leading to occurrence of a difference in degree of focusing.

That is, when the transmitted light image is acquired by irradiating a lung sample with mid-infrared light having a predetermined wavelength (9.6 µm in this example), the cancer tissue is observed as a "blurry" region where a change (variation) in the transmitted light luminance is small, and the normal tissue is observed as a "focused" region where a change in the transmitted light luminance is large. This result is quantified as a focusing measure for each region (pixel) by analysis using a differentiation filter or the like, gradationally divided, and output as an analysis image to enable identification of a tissue which does not depend on a subjective view of an examiner.

Since imaging and analysis are performed at a single wavelength, the measurement time can be made much shorter as compared to a case where hyperspectra are acquired at multiple wavelengths. The measurement time in this example is less than 1 second. The calculation of the focusing measure involves a simple algorithm, does not require processing of a large amount of data unlike cluster analysis, and therefore enables the data processing time to be considerably shortened. The data processing time (time required to display an analysis image on a display unit after acquisition of a measurement image) in this example is less than 1 second. As described above, the method of the present invention enables considerable shortening of the time for measurement and data processing, and can be applied to real-time diagnosis.

### <Example 2: identification of muscular tissue and bone sarcoma>

In Example 1, a lung sample containing a normal tissue and a cancer tissue was used as a test sample (identification target). The identification of a tissue by edge analysis of a mid-infrared light image can also be applied to organs other than the lung. The identification can also be applied to diagnosis of a lesion or the like of a tissue other than cancer. In Example 2, a transmitted light image of mid-infrared light was acquired and edge analysis was performed in the same manner as in Example 1 with the use of a sample containing a bone sarcoma and a muscle tissue that is normal tissue.

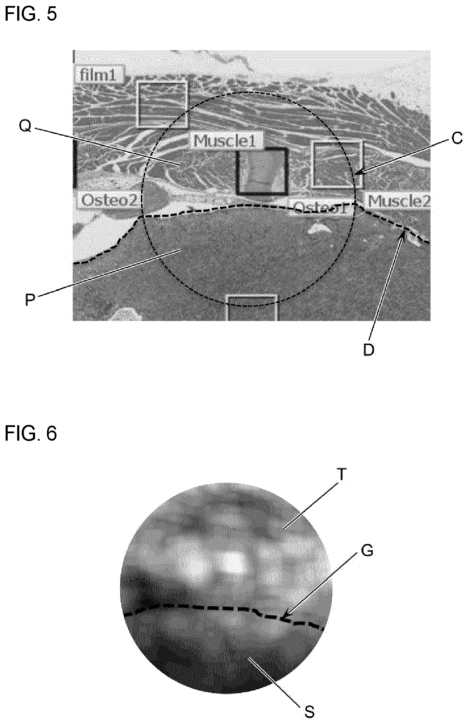

FIG. 5 is a pathological image obtained by staining a sample containing a muscle tissue (normal tissue) and a bone sarcoma, and imaging the sample with an optical microscope. At boundaries between a region P containing a bone sarcoma and a normal tissue region Q, both the tissues are mixed, and therefore these rough boundaries are indicated by a broken line D in FIG. 5. FIG. 6 shows an analysis image obtained by providing a sample (unstained) containing a normal tissue and a bone sarcoma as a sample, irradiating the sample with infrared laser light having a wavelength of 9.86 µm, analyzing a target region from the obtained measurement image, and mapping focusing measures of pixels in 20 gradations. A circular region surrounded by a broken line C in FIG. 5 corresponds to the analysis target region in FIG. 6.

In this example, the minimum and the maximum of the focusing measure in the analysis region of the measurement image were 137 and 199, respectively, and an image was formed with this range divided into 20 gradations. In FIG. 6, a position corresponding to the boundary line D in FIG. 5 is indicated by a broken line G. The focusing measure of the portion marked with a broken line G ranged from 163 to 169, and these values were within 42 to 52% of the region between the minimum and the maximum of the focusing measure. In FIG. 6, a region S (corresponding to the region P in FIG. 5) below the broken line G is a region having a focusing measure smaller than that at the boundary line G, and a region T (corresponding to the region Q in FIG. 5) above the broken line G is a region having a focusing measure larger than that at the boundary line G. This result shows that even in a bone sarcoma sample, the edge analysis of transmitted light image of mid-infrared light can be performed to identify a normal tissue and a bone sarcoma on the basis of a magnitude relationship between a focusing measure calculated by differentiation filter processing and a predetermined threshold.

### <Example 3: Identification of liver cell and cancer>

In Example 1 (lung sample) and Example 2 (bone sarcoma sample) described above, a region having a large focusing measure (high gradation) was a normal tissue, and a region having a small focusing measure (low gradation) was a region including a diseased tissue. On the other hand, depending on the type of biological tissue (organ) or the selected measuring wavelength λ₁, there is a case where a region having a large focusing measure includes a diseased tissue and a region having a small focusing measure is a normal tissue. In Example 3, a transmitted light image of mid-infrared light was acquired and edge analysis was performed in the same manner as in Examples 1 and 2 with the use of a liver sample containing cancer, a normal tissue and a lymphocyte (normal tissue).

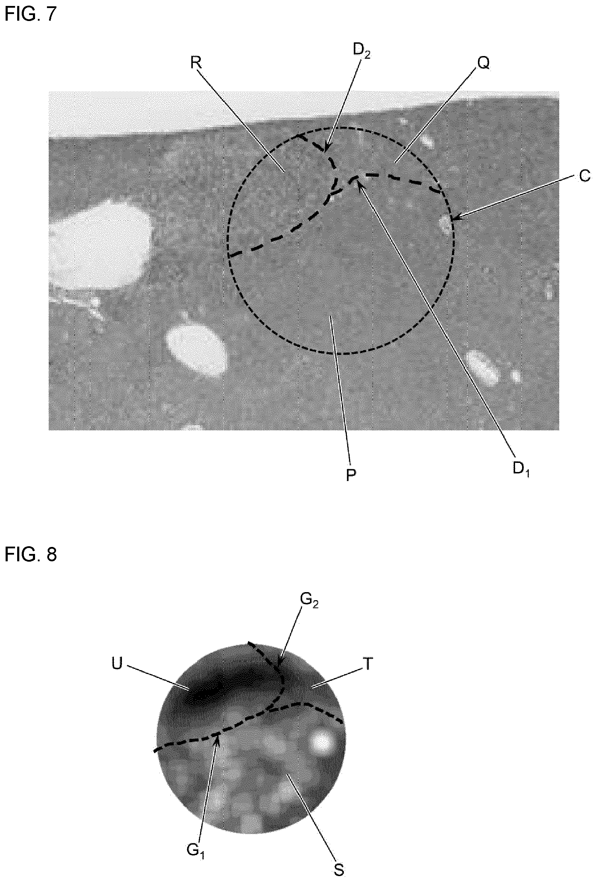

FIG. 7 is a pathological image obtained by staining a liver sample containing cancer, a normal tissue and a lymphocyte, and imaging the sample with an optical microscope. In a circle surrounded by a broken line C in the drawing, boundaries of regions of the tissues are indicated by broken lines D₁ and D₂. FIG. 8 shows an analysis image obtained by providing a liver sample (unstained) containing cancer, a normal tissue and a lymphocyte as a sample, irradiating the sample with infrared laser light having a wavelength of 9.3 µm, analyzing a target region from the obtained measurement image, and mapping focusing measures of pixels in 20 gradations.

In this example, the minimum and the maximum of the focusing measure in the analysis region of the measurement image were 133 and 163, respectively, and an image was formed with this range divided into 20 gradations. In Fig. 8, rough boundaries of the tissues are indicated by broken lines G₁ and G₂. The focusing measure of the region including cancer and the region of the normal tissue ranged from 145 to 148, and these values were within 42 to 52% of the region between the minimum and the maximum of the focusing measure. The focusing measure of the boundary between the region including cancer and the region of lymphocyte was also within the range of 145 to 148. On the other hand, in the analysis image of FIG. 8, there was no difference in gradation at the boundary G₂ between the region of the normal tissue and the region of the lymphocyte.

In this Example, contrary to Examples 1 and 2, a region having a gradation higher than the boundary gradation and a larger focusing measure was a region including a diseased tissue (cancer), and a region having a gradation lower than the boundary gradation and a smaller focusing measure was a normal tissue.

For each of Examples 1 to 3, the measurement sample, the measurement wavelength λ₁, the range of the focusing measure (maximum and minimum), and the range of the focusing measure at the boundary between the diseased tissue and the normal tissue are listed in Table 1.

**[Table 1]**

| | Sample (diseased species) | Measuring wavelength λ₁ (µm) | Focusing measure | | |
|---|---|---|---|---|---|
| | | | Minimum | Maximum | Boundary |
| Example 1 | Lung (cancer) | 9.6 | 130 | 187 | 157-166 |
| Example 2 | Muscle (bone sarcoma) | 9.86 | 137 | 199 | 163-169 |
| Example 3 | Liver (cancer) | 9.3 | 133 | 163 | 145-148 |

### <Application Example>

As described above, a wavelength at which a difference in absorbance is large may be selected as the measuring wavelength λ₁ according to the type of tissue (organ) to be measured, and by appropriately selecting a measuring wavelength λ₁ and performing measurement and analysis, a biological tissue can be identified on the basis of the focusing measure even when the biological tissue is a tissue other than that shown in the above examples.

When the organ is unknown or when the measuring wavelength most suitable for the organ to be identified is unknown, measurement and analysis are performed at a plurality of wavelengths after focusing adjustment is performed, and a result of measurement at a wavelength at which a difference between the maximum and the minimum of the focusing measure is small is adopted. The results of measurement and analysis at a plurality of wavelengths may be compared with a database to identify an organ or a lesion type. By accumulating matching data of analysis images with pathological images for identifying a diseased tissue and a normal tissue and compiling a database, application to automation of identification by artificial intelligence can be expected. Information related to a threshold of a focus measure for identifying a tissue, and the like may be stored in the database for analysis results. Examples of the diseased tissue to be analyzed include non-cancer tissues such as tissues affected by muscle infarction, fatty liver and liver cirrhosis, and amyloid deposited glomeruli in kidney tissues, in addition to cancer.

In the above-described example, laser light that is coherent light is used as measurement light. When the measurement light is coherent light having a single wavelength λ₁, light having a wavelength λ₁ is imaged by the imaging element, and imaging and analysis are performed at a single wavelength, so that the measurement and analysis time can be made shorter as compared to a case where imaging and analysis are performed at multiple wavelengths (e.g. a case where a hyperspectrum is acquired).

### (Use of incoherent light)

As described above, the measurement light may be incoherent light including light having a plurality of wavelengths. When the measurement light is incoherent light, placement of a spectroscopic unit such as a wavelength filter or a spectrometer between the light source 11 and the sample S or/and between the sample S and the imaging element 12 enables the imaging element to image only a part of multi-wavelength light included in a light source, which has a specific wavelength λ₁, so that analysis can be performed as in the case where the measurement light is coherent light. When incoherent light is used, focusing adjustment with light having a wavelength λ₀ and measurement with light having a wavelength λ₁ can be performed using the same light source when the wavelength of light reaching the imaging element is changed by replacement of a wavelength filter or adjustment of a spectrometer.

### (Tiling)

Although edge analysis of a measurement image obtained by one measurement was performed, and a tissue was identified on the basis of the calculated focusing measure in the above-described example, a plurality of measurement results may be combined to perform image analysis and identification of a tissue. For example, the sample stage 13 is moved in x and y directions, different portions of the sample S are irradiated with laser light to acquire measurement images, and a plurality of measurement images are combined (tiled) to obtain a measurement image of a wider region of the sample.

By performing edge analysis of the measurement image obtained by the tiling and calculating the focusing measure, a tissue can be identified over a wider range as compared to one measurement. Edge analysis may be performed to form an analysis image for each measurement, followed by combining (tiling) a plurality of analysis images. In tiling of a plurality of measurement images and/or analysis images, various algorithms which are used for tiling of images photographed under an optical microscope and an electron microscope may be applied.

### DESCRIPTION OF REFERENCE SIGNS

- 1: Biological tissue identification apparatus
- 11: Light source
- L₀, L₁: Measurement light (mid-infrared light)
- 12: Imaging element
- 13: Sample stage
- S: Sample
- 21,23: Optical element
- 30: Computer
- 31: Control unit
- 311: Light adjustment unit
- 313: Position adjustment unit
- 315: Calculation analysis unit
- 317: Image forming unit
- 33: Display unit
- 35: Storage unit
- 37: Input unit

## Claims

1. A biological tissue identification apparatus (1) comprising:
a light source (11) capable of irradiating a predetermined region of a biological tissue with a measurement light that includes mid-infrared light;
an imaging element (12, 19) for acquiring a measurement image by imaging a part of the measurement light transmitted through the biological tissue or a part of the measurement light reflected at the biological tissue, which has a specific wavelength λ₁ being a wavelength in a range of 2 µm to 20 µm; and
a computer (30) including a calculation analysis unit (315) capable of calculating focusing measures in a plurality of regions of the measurement image by an edge analysis of the measurement image, and the calculation analysis unit (315) being configured to identify the biological tissue based on the focusing measures.

2. The biological tissue identification apparatus (1) according to claim 1, wherein the calculation analysis unit (315) calculates the focusing measures by subjecting the measurement image to differentiation filtering.

3. The biological tissue identification apparatus (1) according to claim 1 or 2, wherein in the calculation analysis unit (315), whether or not the biological tissue is a normal tissue is identified based on a magnitude relationship between the focusing measure and a predetermined threshold.

4. The biological tissue identification apparatus (1) according to any one of claims 1 to 3, wherein the computer (30) includes an image forming unit (317) for mapping the focusing measures to form an analysis image.

5. The biological tissue identification apparatus (1) according to any one of claims 1 to 4, wherein the light source (11) is a laser light source capable of scanning wavelengths.

6. The biological tissue identification apparatus (1) according to any one of claims 1 to 5, wherein the light source (11) is a quantum cascade laser or a laser including an optical parametric oscillator

7. The biological tissue identification apparatus (1) according to any one of claims 1 to 6, wherein a program for causing the calculation analysis unit to execute processing in which the measurement image acquired by the imaging element (12, 19) is subjected to edge analysis to calculate focusing measures in a plurality of regions of the measurement image is stored in a storage unit (35) of the computer (30).

8. A method for identifying a biological tissue using the apparatus (1) set forth in any one of claims 1 to 7,
the method comprising:
irradiating a predetermined region of a biological tissue with a measurement light from the light source (11) that includes mid-infrared light;
acquiring a measurement image (S5) by imaging a part of measurement light transmitted through the biological tissue or a part of the measurement light reflected at the biological tissue, which has a specific wavelength λ₁ in a range of 2 µm to 20 µm;
calculating focusing measures (S6) in a plurality of regions of the measurement image by an edge analysis of the measurement image; and
identifying the biological tissue based on the focusing measures.

9. The method according to claim 8, wherein the edge analysis is performed by differentiation filtering of the measurement image.

10. The method for identifying a biological tissue according to claim 8 or 9, wherein the biological tissue is irradiated with laser light having a wavelength λ₁ as the measurement light, optionally
wherein the measurement light is quantum cascade laser light or laser light amplified by optical parametric oscillation.

11. The method according to any one of claim 8 to 10, wherein focusing adjustment (S2) is performed before the measurement image is acquired.

12. The method according to claim 11, wherein in the focusing adjustment (S2)
a predetermined region of the biological tissue is irradiated with the measurement light that includes mid-infrared light,
a focusing adjusting image (A₁) is acquired (S24) by imaging light having wavelength λ₀ in a range of 2 µm to 20 µm, which is different from the wavelength λ₁, and
a position of a sample containing the biological tissue and/or a position of another optical element is adjusted (S28) so that a degree of focusing of the focusing adjusting image increases.

13. The method according to claim 12, wherein in the focusing adjustment (S2) the biological tissue is irradiated with laser light having a wavelength λ₀.

14. The method according to any one of claims 8 to 13, wherein whether or not the biological tissue is a normal tissue is identified based on a magnitude relationship between the focusing measure and a predetermined threshold.

15. The method for identifying a biological tissue according to any one of claims 8 to 14, wherein the method is configured to identify whether or not the biological tissue contains cancer.

## Patentansprüche

1. Identifikationseinrichtung (1) für biologisches Gewebe, umfassend:
eine Lichtquelle (11), die in der Lage ist, eine vorbestimmte Region eines biologischen Gewebes mit einem Messlicht zu bestrahlen, das Licht im mittleren Infrarot einschließt;
ein Abbildungselement (12, 19) zum Erfassen eines Messbilds durch Abbilden eines Teils des Messlichts, das durch das biologische Gewebe hindurch übertragen wurde, oder eines Teils des Messlichts, das an dem biologischen Gewebe reflektiert wurde, das eine spezifische Wellenlänge *λ*₁ aufweist, die eine Wellenlänge in einem Bereich von 2 µm bis 20 µm ist; und
einen Computer (30), einschließlich einer Berechnungsanalyseeinheit (315), die in der Lage ist, Fokussierungsmaßnahmen in einer Vielzahl von Regionen des Messbilds durch eine Randanalyse des Messbilds zu berechnen, und die Berechnungsanalyseeinheit (315) konfiguriert ist, um das biologische Gewebe auf Grundlage der Fokussierungsmaßnahmen zu identifizieren.

2. Identifikationseinrichtung (1) für biologisches Gewebe nach Anspruch 1, wobei die Berechnungsanalyseeinheit (315) die Fokussierungsmaßnahmen berechnet, indem das Messbild einem Differenzierungsfiltern unterzogen wird.

3. Identifikationseinrichtung (1) für biologisches Gewebe nach Anspruch 1 oder 2, wobei in der Berechnungsanalyseeinheit (315)
auf Grundlage einer Größenordnungsbeziehung zwischen der Fokussierungsmaßnahme und einem vorbestimmten Schwellenwert identifiziert wird, ob das biologische Gewebe ein normales Gewebe ist oder nicht.

4. Identifikationseinrichtung (1) für biologisches Gewebe nach einem der Ansprüche 1 bis 3, wobei der Computer (30) eine Bildbildungseinheit (317) zur Darstellung der Fokussierungsmaßnahmen einschließt, um ein Analysebild zu bilden.

5. Identifikationseinrichtung (1) für biologisches Gewebe nach einem der Ansprüche 1 bis 4, wobei die Lichtquelle (11) eine Laserlichtquelle ist, die in der Lage ist, Wellenlängen abzutasten.

6. Identifikationseinrichtung (1) für biologisches Gewebe nach einem der Ansprüche 1 bis 5, wobei die Lichtquelle (11) ein Quantenkaskadenlaser oder ein Laser ist, der einen optischen parametrischen Oszillator einschließt.

7. Identifikationseinrichtung (1) für biologisches Gewebe nach einem der Ansprüche 1 bis 6, wobei ein Programm zum Veranlassen der Berechnungsanalyseeinheit, Verarbeitung auszuführen, bei der das durch das Abbildungselement (12,19) erfasste Messbild Randanalyse unterzogen wird, um Fokussierungsmaßnahmen in einer Vielzahl von Regionen des Messbilds zu berechnen, in einer Speichereinheit (35) des Computers (30) gespeichert ist.

8. Verfahren zum Identifizieren eines biologischen Gewebes unter Verwendung der in einem der Ansprüche 1 bis 7 dargelegten Einrichtung (1),
wobei das Verfahren umfasst:
Bestrahlen einer vorbestimmten Region eines biologischen Gewebes mit einem Messlicht aus der Lichtquelle (11), das Licht im mittleren Infrarot einschließt;
Erfassen eines Messbilds (S5) durch Abbilden eines Teils des Messlichts, das durch das biologische Gewebe hindurch übertragen wurde, oder eines Teils des Messlichts, das an dem biologischen Gewebe reflektiert wurde, das eine spezifische Wellenlänge *λ*₁ in einem Bereich von 2 µm bis 20 µm aufweist;
Berechnen von Fokussierungsmaßnahmen (S6) in einer Vielzahl von Regionen des Messbilds durch eine Randanalyse des Messbilds; und
Identifizieren des biologischen Gewebes auf Grundlage der Fokussierungsmaßnahmen.

9. Verfahren nach Anspruch 8, wobei die Randanalyse durch Differenzierungsfiltern des Messbilds durchgeführt wird.

10. Verfahren zum Identifizieren eines biologischen Gewebes nach Anspruch 8 oder 9, wobei das biologische Gewebe mit Laserlicht mit einer Wellenlänge *λ*₁, als dem Messlicht bestrahlt wird,
wobei optional das Messlicht Quantenkaskadenlaserlicht oder Laserlicht ist, das durch optische parametrische Oszillation amplifiziert wurde.

11. Verfahren nach einem der Ansprüche 8 bis 10, wobei Fokussierungsanpassung (S2) durchgeführt wird, bevor das Messbild erfasst wird.

12. Verfahren nach Anspruch 11, wobei bei der Fokussierungsanpassung (S2) eine vorbestimmte Region des biologischen Gewebes mit dem Messlicht, das Licht im mittleren Infrarot einschließt, bestrahlt wird,
ein Fokussierungsanpassungsbild (A₁) durch Abbilden von Licht mit Wellenlänge *λ*₀ in einem Bereich von 2 µm bis 20 µm erfasst (S24) wird, die sich von der Wellenlänge *λ*₁ unterscheidet, und
eine Position einer Probe, die das biologische Gewebe enthält, und/oder eine Position eines anderen optischen Elements so angepasst (S28) wird, dass ein Fokussierungsgrad des Fokussierungsanpassungsbilds zunimmt.

13. Verfahren nach Anspruch 12, wobei bei der Fokussierungsanpassung (S2) das biologische Gewebe mit Laserlicht mit einer Wellenlänge *λ*₀ bestrahlt wird.

14. Verfahren nach einem der Ansprüche 8 bis 13, wobei auf Grundlage einer Größenordnungsbeziehung zwischen der Fokussierungsmaßnahme und einem vorbestimmten Schwellenwert identifiziert wird, ob das biologische Gewebe ein normales Gewebe ist oder nicht.

15. Verfahren zum Identifizieren eines biologischen Gewebes nach einem der Ansprüche 8 bis 14, wobei das Verfahren konfiguriert ist, um zu identifizieren, ob das biologische Gewebe Krebs enthält oder nicht.

## Revendications

1. Appareil d'identification de tissu biologique (1) comprenant :
une source de lumière (11) capable d'irradier une région prédéterminée d'un tissu biologique avec une lumière de mesure qui inclut une lumière dans l'infrarouge moyen ;
un élément d'imagerie (12, 19) pour acquérir une image de mesure en prenant une image d'une partie de la lumière de mesure transmise à travers le tissu biologique ou d'une partie de la lumière de mesure réfléchie par le tissu biologique, qui présente une longueur d'onde *λ*₁ spécifique qui est une longueur d'onde dans une plage de 2 µm à 20 µm ; et
un ordinateur (30) incluant une unité d'analyse par calcul (315) capable de calculer des mesures de focalisation dans une pluralité de régions de l'image de mesure par une analyse de contours de l'image de mesure, et l'unité d'analyse par calcul (315) étant configurée pour identifier le tissu biologique sur la base des mesures de focalisation.

2. Appareil d'identification de tissu biologique (1) selon la revendication 1, dans lequel l'unité d'analyse par calcul (315) calcule les mesures de focalisation en soumettant l'image de mesure à un filtrage de différenciation.

3. Appareil d'identification de tissu biologique (1) selon la revendication 1 ou 2, dans lequel dans l'unité d'analyse par calcul (315),
le fait que le tissu biologique soit ou non un tissu normal est identifié sur la base d'une relation d'amplitude entre la mesure de focalisation et un seuil prédéterminé.

4. Appareil d'identification de tissu biologique (1) selon l'une quelconque des revendications 1 à 3, dans lequel l'ordinateur (30) inclut une unité de formation d'image (317) pour mapper les mesures de focalisation pour former une image d'analyse.

5. Appareil d'identification de tissu biologique (1) selon l'une quelconque des revendications 1 à 4, dans lequel la source de lumière (11) est une source de lumière laser capable de balayer des longueurs d'onde.

6. Appareil d'identification de tissu biologique (1) selon l'une quelconque des revendications 1 à 5, dans lequel la source de lumière (11) est un laser à cascade quantique ou un laser incluant un oscillateur paramétrique optique.

7. Appareil d'identification de tissu biologique (1) selon l'une quelconque des revendications 1 à 6, dans lequel un programme pour amener l'unité d'analyse par calcul à exécuter un traitement dans lequel l'image de mesure acquise par l'élément d'imagerie (12, 19) est soumise à une analyse de contours pour calculer des mesures de focalisation dans une pluralité de régions de l'image de mesure est stocké dans une unité de stockage (35) de l'ordinateur (30).

8. Procédé d'identification d'un tissu biologique utilisant l'appareil (1) présenté dans l'une quelconque des revendications 1 à 7,
le procédé comprenant les étapes consistant à :
irradier une région prédéterminée d'un tissu biologique avec une lumière de mesure provenant de la source de lumière (11) qui inclut une lumière dans l'infrarouge moyen ;
acquérir une image de mesure (S5) en prenant une image d'une partie de la lumière de mesure transmise à travers le tissu biologique ou d'une partie de la lumière de mesure réfléchie par le tissu biologique, qui présente une longueur d'onde *λ*₁ spécifique dans une plage de 2 µm à 20 µm ;
calculer des mesures de focalisation (S6) dans une pluralité de régions de l'image de mesure par une analyse de contours de l'image de mesure ; et
identifier le tissu biologique sur la base des mesures de focalisation.

9. Procédé selon la revendication 8, dans lequel l'analyse de contours est effectuée par un filtrage de différenciation de l'image de mesure.

10. Procédé d'identification d'un tissu biologique selon la revendication 8 ou 9, dans lequel le tissu biologique est irradié avec une lumière laser présentant une longueur d'onde *λ*₁ en tant que lumière de mesure, facultativement
dans lequel la lumière de mesure est une lumière laser à cascade quantique ou une lumière laser amplifiée par une oscillation paramétrique optique.

11. Procédé selon l'une quelconque des revendications 8 à 10, dans lequel un ajustement de focalisation (S2) est effectué avant l'acquisition de l'image de mesure.

12. Procédé selon la revendication 11, dans lequel, lors de l'ajustement de focalisation (S2), une région prédéterminée du tissu biologique est irradiée avec la lumière de mesure qui inclut une lumière dans l'infrarouge moyen,
une image d'ajustement de focalisation (A₁) est acquise (S24) en prenant une image de la lumière présentant une longueur d'onde *λ*₀ dans une plage de 2 µm à 20 µm, qui est différente de la longueur d'onde *λ*₁, et
une position d'un échantillon contenant le tissu biologique et/ou une position d'un autre élément optique est ajustée (S28) de sorte qu'un degré de focalisation de l'image d'ajustement de focalisation augmente.

13. Procédé selon la revendication 12, dans lequel, lors de l'ajustement de focalisation (S2), le tissu biologique est irradié avec une lumière laser présentant une longueur d'onde *λ*₀.

14. Procédé selon l'une quelconque des revendications 8 à 13, dans lequel le fait que le tissu biologique soit ou non un tissu normal est identifié sur la base d'une relation d'amplitude entre la mesure de focalisation et un seuil prédéterminé.

15. Procédé d'identification d'un tissu biologique selon l'une quelconque des revendications 8 à 14, dans lequel le procédé est configuré pour identifier si le tissu biologique contient ou non un cancer.
